# EUROPEAN PATENT SPECIFICATION

(11) **EP 1 346 746 B1**
(45) Date of publication and mention of the grant of the patent: **11.10.2006**
(21) Application number: 02076050.0
(22) Date of filing: 18.03.2002
(51) Int. Cl.: A61M 25/01

(54) **Medical device for gripping an elongated member**
Medizinisches Gerät zum Greifen eines langgestreckten Elements
Instrument médical pour la saisie d'un membre allongé

(43) Date of publication of application: 24.09.2003
(73) Proprietor: WILLIAM COOK EUROPE ApS, 4632 Bjaeverskov (DK); Cook Incorporated, Bloomington, Indiana 47402-0489 (US)
(72) Inventor: Christensen, Jess, 4130 Viby Sjaelland (DK); Hendriksen, Per, 4171 Glumsoe (DK); Moller, Johnny, 4130 Viby Sjaeland (DK)
(74) Representative: Jehan, Robert

(56) References cited:
- US-A- 5 161 534
- US-A- 5 392 778
- US-A- 5 423 331
- US-A- 5 634 475

## Description

The present invention relates to the field of medical devices in general, and more particularly to methods and devices for the manipulation of elongated members, such as guidewires, during access to animal or human bodily passages such as blood vessels.

Medical guidewires have a small diameter, making it impractical for a physician to grip and manipulate such a guidewire directly by hand. This is a major disadvantage in an emergency where it is critical to the survival of patients that catheterisation of branched or convoluted blood vessels be an efficient procedure. The present invention concerns a vice, which is also known as a "torque" device, a "gripping" device, a "steering" device or "migration controller" device, and which is used to facilitate the manipulation of small diameter guidewires.

The gripping means described in the prior art are broadly divided into systems either using a rubber, or a mechanical mechanism such as the one disclosed in US patents 5,851,189 and 5,392,778.

US patent 5,851,189 to Forber discloses a torque device for attaching to and selectively gripping and releasing a guidewire to permit rotational and longitudinal manipulation of the guidewire and thereby to steer the guidewire through the vessels of a patient. The grip of the device over the guidewire is adjustable by means of two interconnected cylinders that can rotate with respect to each other. The first cylinder is the gripping device comprising a spindle with two fingers straddling the guidewire. The second cylinder is a cap that is rotated with respect to the first cylinder to force the distal end of the fingers to advance along a tapering bore in the cap and to close and grip the guidewire. The Forber device is simple and the guidewire is kept centred between the fingers of the spindle because the cap has a cylindrical bore through which the guidewire is threaded and maintained within the axis of the device. Furthermore, in the preferred embodiment of the Forber device, the spindle has only two fingers, so that the device can accommodate the full range of guidewire diameters with one single device. However, the Forber device has two main disadvantages. Firstly its gripping ability depends on the action of a spindle with only two fingers, and secondly, because the cap that maintains the guidewire centred within the spindle is an integral part of the gripping mechanism, manual access to the cap must be preserved at all times.

US patent 5,392,778 to Horzewski discloses a similar device consisting of two cylinders that slide with respect to each other, one surrounding the distal part of the other. The inner cylinder comprises a multitude of prongs that encircle a guidewire that is threaded through a central passage of the inner cylinder. The outer cylinder, which comprises an inner ring encircling the prongs, can be advanced manually with respect to the first cylinder to close the prongs over the guidewire and provide a grip when the device is used to manipulate the guidewire. The Horzewski device has substantially the same disadvantages as the Forber device. The prongs are made of a resilient material, permitting the prongs to bend during compression by the outer cylinder, possibly compromising the gripping ability of the device. In addition, manual access to the outer cylinder that maintains the guidewire centred within the device has to be preserved.

US 5,634,475 discloses a guidewire delivery assist device including a cylindrical body, having a lumen extending axially therethrough from a proximal end to a distal end thereof, for receiving a guidewire, and an integral grip. The grip includes a plurality of flexible grip members extending longitudinallythrough at least a portion of the cylindrical body and forming a plurality of internal surface portions defining a portion of the lumen of the cylindrical body. The grip members and interior surface portions are flexibly collapsible in a radial direction of the cylindrical body and a guidewire resident in the lumen of the cylindrical body is frictionally engageable by the internal surface portions.

One of the major disadvantages of the prior art devices is the possibility, during introduction of the guidewire, that it might get caught in the linear slits between the fingers or prongs of the device, causing the guidewire to become entrapped in the device, which could lead to kinking of the guidewire requiring it to be removed and replaced. Time might therefore be wasted, particularly in a situation where it is critical for the survival of a patient that a guidewire or other elongated member be introduced promptly in the vascular system.

The present invention seeks to provide a device that can easily grip an elongated member such as a guidewire, that is self-centring, meaning that an elongated member could be maintained in the centre of the device without the help of any other component, that has an excellent ability to grip, that is capable of accommodating a wide range of elongated member's diameters, and that is yet simple and inexpensive to manufacture.

The foregoing problems are solved and a technical advance is achieved in a medical device, such as a vice for gripping an elongated member, the vice comprising a plurality of fingers encircling the elongated member when it is threaded through the central passage of the device. When the fingers are urged together into an engagement position, a portion of each finger grips the elongated member. Each finger comprises a pair of side surfaces, each of which is to be urged towards a side surface of an adjacent finger. Adjacent side surfaces are shaped with respect to each other, resulting in spaces that are not straight and thereby preventing entrapment of the elongated member between the side surfaces of the fingers of the vice.

A vice formed in accordance with the present invention has a number of advantages. Since the side surfaces of adjacent fingers are shaped, and therefore the space or slit between the fingers is not straight, the elongated member is prevented to penetrate into the spaces or slits between the fingers, therefore avoiding one of the disadvantages of prior art devices where a guidewire could become entrapped between fingers or prongs having a straight space between them. A guidewire is, despite its high flexibility, to be regarded as straight on portions as short as the shaped part of the vice. The adjacent side surfaces of a pair of adjacent fingers could be shaped with all kinds of recesses and substantially complementary protrusions, thereby preventing penetration of the slits by a substantially straight elongated member. Kinking of the guidewire is thus totally avoided by the present invention. A further advantage of the vice is the self-centring ability: because the elongated member is unable to become entrapped in the slits between fingers, it is therefore kept in the central passage of the vice without the use of any other component of the vice. A device according to the present invention will accommodate a wide range of diameters and/or shapes of the elongated member, since even an elongated member having a diameter much smaller than the distance between the side surfaces of adjacent fingers would not be able to penetrate a slit that is not straight.

Embodiments of the present invention will now be described by way of example and with reference to the accompanying drawings in which:
Figure 1 is a three-dimensional view illustrating the preferred embodiment of the vice in accordance with the present invention; and
Figure 2 is a lateral view of the vice of Figure 1 in accordance with the present invention; and
Figure 3 is an end on view of the distal end of the vice of Figure 1 in accordance with the present invention; and
Figure 4 illustrates a longitudinal cross-sectional view of the vice at level A-A of Figure 3, in accordance with the present invention; and
Figure 5 is a three-dimensional view illustrating the inner side of a finger of the vice, in accordance with the present invention; and
Figure 6 is a three-dimensional view illustrating a second embodiment of the vice in accordance with the present invention; and
Figure 7 is a three-dimensional view illustrating a third embodiment of the vice in accordance with the present invention; and
Figure 8 is a longitudinal cross-sectional view of a vice in accordance with the present invention illustrating features of adjacent side surfaces of adjacent fingers.

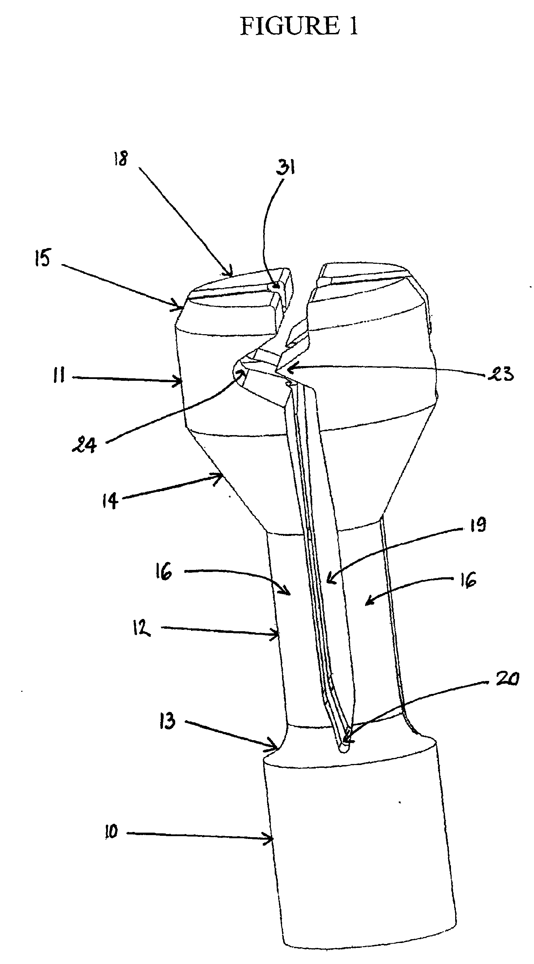

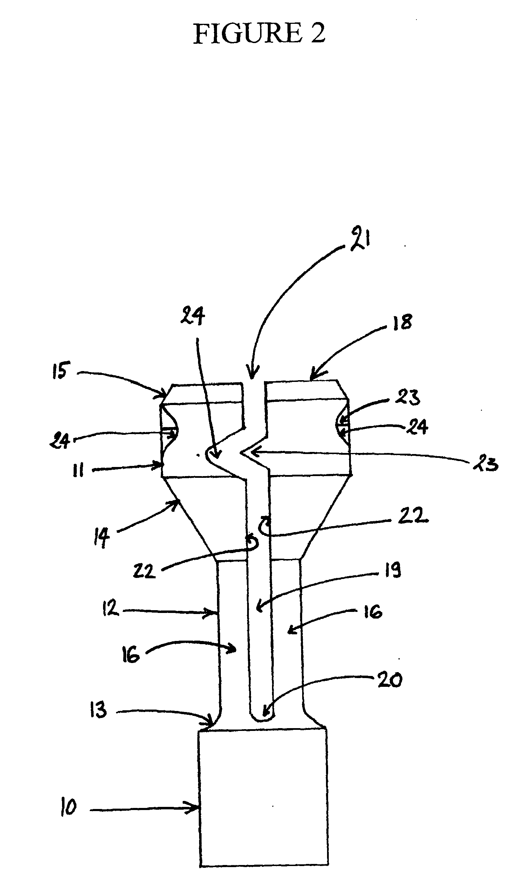

Figures 1 to 5 illustrate the preferred embodiment of the vice, which comprises three main parts: a base **10**, a neck **12** and a head **11.** Base **10** is preferably cylindrical and located in the proximal portion of the device. Neck **12** is preferably cylindrical and located between base **10** and head **11**. Head **11**, which is also preferably cylindrical, is located in the distal portion of the device. Proximal intermediate part **13** extends between neck **12** and base **10**, and distal intermediate part **14** extends between neck **12** and head **11.** The proximal intermediate part **13**, which can have any shape, preferably tapers from the larger diameter base **10** to the smaller diameter neck **12**. Alternatively, the diameter of neck **12** can be made equal to or larger than the diameter of head **11** and/or base **10**. The distal intermediate part **14** has preferably a conical shape, but can have any other shape. Distal part **15** of the vice is preferably conical and has a diameter that decreases towards the distal end **18** of the device. Alternatively, distal part **15** can have any shape and be smaller than, equal to or larger than head **11**.

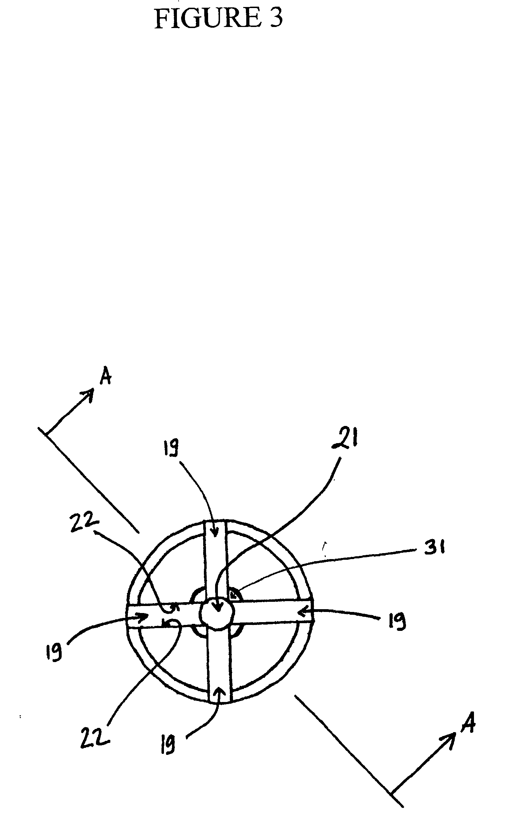

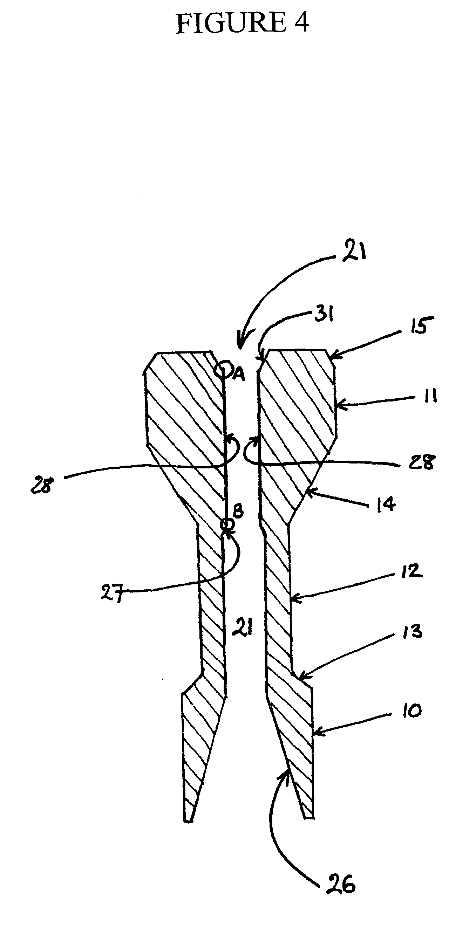

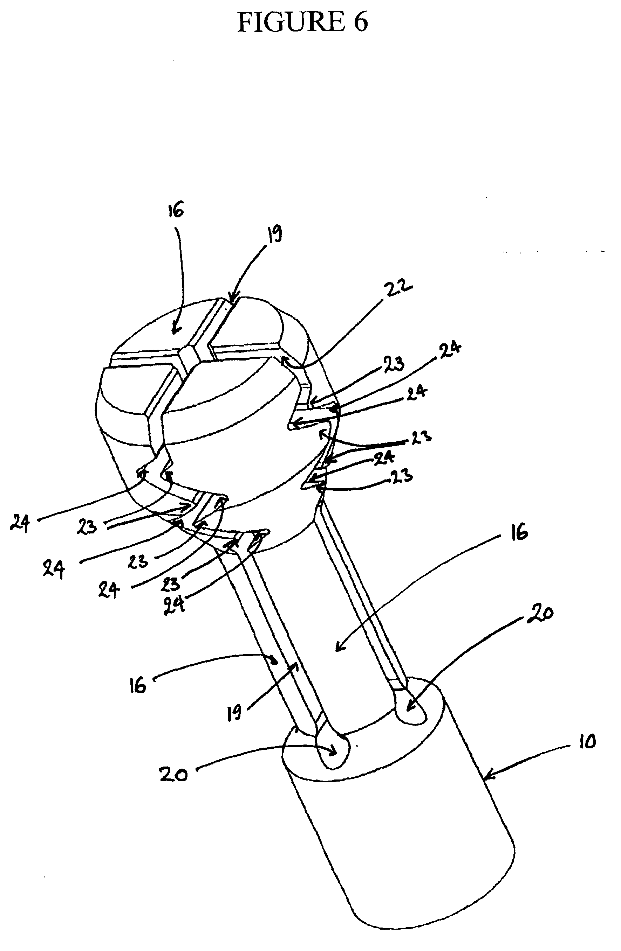

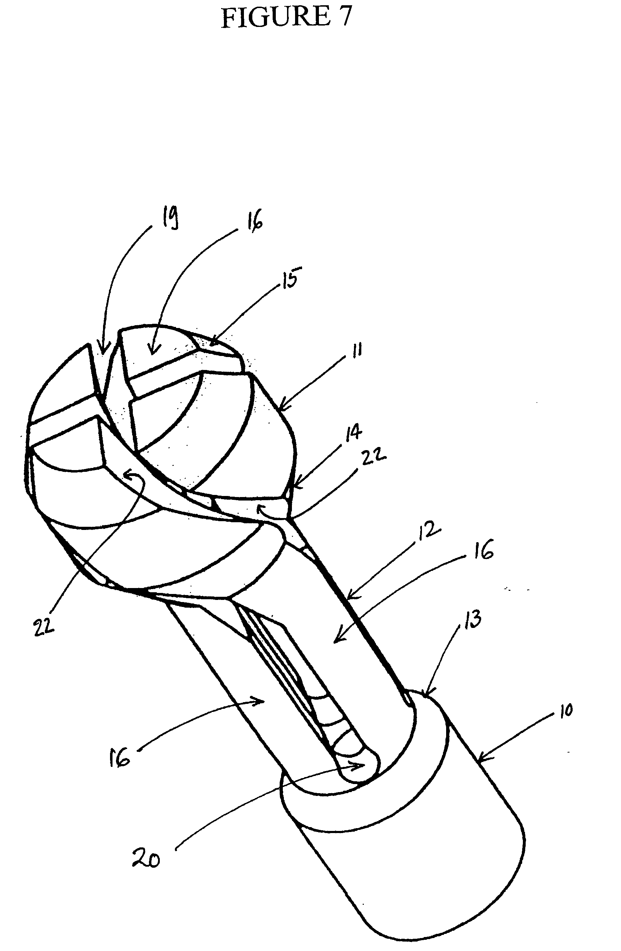

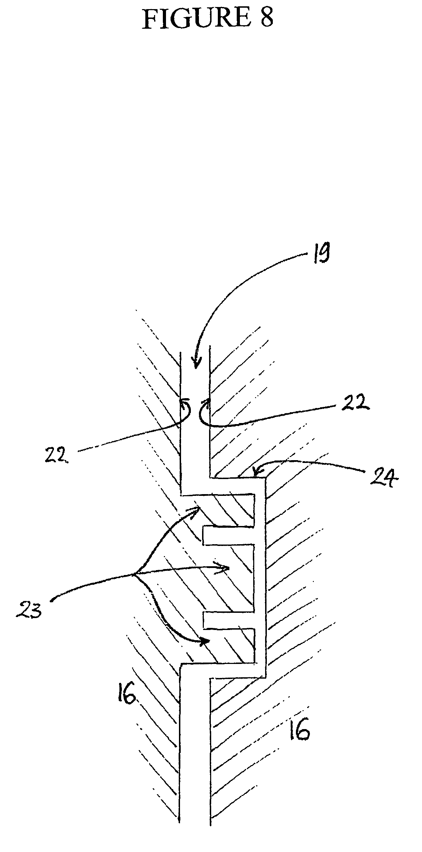

A vice is used for gripping elongated members such as a guidewire. The elongated member is threaded within a central passage **21** of the vice, as illustrated in Figure 4. In Figures 3 to 5, the distal opening of the central passage **21** is illustrated as an enlarged distal conical bore **31**, located within the distal part **15** of the vice, in order to facilitate the introduction of an elongated member through the distal opening of the central passage. It should be noted, however, that it is preferable to introduce the elongated member into the vice through the proximal conical bore **26**. The part of the vice distal to base **10** comprises at least two fingers, preferably three fingers, and most preferably four fingers **16**, each extending longitudinally from base **10** and each having a free distal end **18**. It is to be noted that although a three-finger device provides a self-centring mechanism for the elongated member, the preferred embodiment of the present invention comprises four fingers because of the ease of manufacturing such a device. The gripping action of the device is achieved when the fingers **16** are urged together into an engagement position with the elongated member. The space between two adjacent side surfaces **22** of two adjacent fingers **16** delimits a slit **19** having a shape that is not straight, for preventing access of the elongated member therethrough. The number of slits between fingers **16** depends on the total number of fingers comprised in the device; for example, a vice with four fingers has four slits. The proximal part **20** of slit **19**, located at the level of proximal intermediate part **13**, has a shape that is for example triangular, as illustrated in Figure 1, or rounded as illustrated in Figures 6 and 7. Proximal part **20** can also have any other shape. Because elongated members are prone to become entrapped between the fingers of a vice having straight slits, the vice of the present invention has slits that are not straight in shape. Features of the side surfaces **22**, such as for example protuberance **23** and recess **24**, are responsible for creating non-straight slits. The parts of the slit where the side surface features are located constitute the non-straight part of the slit. This non-straight part can extend over the entire length of the slit or preferably only over a fraction of its length. The non-straight part of the slit is preferably located in the distal portion of the vice, more preferably at the level of the distal intermediate part **14** and head **11**, and most preferably at the level of the head **11** of the vice, as a single non-straight part. A single slit can also have multiple non-straight parts that are not contiguous. Figure 6 is an example of a vice wherein the non-straight part of the slit is located in both the distal intermediate part **14** and the head **11**. A vice formed with non-straight slits, in accordance with the present invention, makes it impossible for an elongated member to become entrapped between the fingers of the device. An exemplary feature of the side surface **22** consists of a protuberance **23** preferably located at the level of head **11** of one side surface **22** of a finger **16**, which substantially complements a recess **24** on the adjacent side surface **22** of an adjacent finger **16**, when the fingers are urged together in an engagement position. In the preferred embodiment, which comprises four fingers, the slits **19** that are diametrically opposed are identical. One of the main advantages of such an embodiment is that the manufacturing process is simplified and efficient, as it is possible, for example during electro-discharge machinery cutting, to cut in a single pass a pair of shaped and diametrically opposed slits. A second cut creates the other pair of shaped opposed slits, which have either an identical or a different shape than that of the former pair of slits. Therefore, the fingers of the vice of the preferred embodiment are all different. If opposing slits are not cut simultaneously, i.e. four cuts are then necessary to create four slits. In the latter case, two different pairs of identical fingers or four identical fingers are created. Alternative devices according to the present invention comprise fingers with identical or dissimilar side surfaces. For example, a finger can have a protuberance or a recess on both of its side surfaces. A protuberance or recess of one side surface may be offset longitudinally with respect to the protuberance or recess of the other side surface of the same finger. Further devices in accordance with the present invention comprise fingers having features on adjacent side surfaces of adjacent fingers that are not complementary. Figure 8 illustrates one non-limitative example of non-complementary features. Producing a vice with complementary features of adjacent side surfaces has a major advantage with respect to ease of manufacture compared to production of a vice with non-complementary features, since in the former case, cutting of a slit results automatically in the appropriate shaping of the adjacent complementary features. This is not the case for side surfaces with non-complementary features, where multiple cuts are needed to create a slit.

Figure 4 illustrates a longitudinal cross-scctional view of the vice of Figure 3 at level A-A. Central passage **21** has three portions: a proximal portion that corresponds to base **10**, an intermediate portion that corresponds to proximal intermediate part **13** and neck **12**, and a distal portion that corresponds to distal intermediate part **14**, head **11** and distal part **15**. The proximal portion of the central passage **21** has a proximal bore **26**, that is preferably conical, to facilitate introduction of an elongated member in a proximal to distal direction. The distal portion of passage **21** within the distal part **15** of the vice is preferably conical to allow for introduction of an elongated member in a distal to proximal direction. The proximal conical bore **26** has sloping edges, preferably at an angle of about 15° with respect to the longitudinal axis of the vice. Central passage **21**, within neck **12** of the vice, is preferably cylindrical and has a diameter that is slightly larger than the diameter of the central passage **21** within head **11**. Inner bore **27**, at the junction of intermediate portion and distal portions of central passage **21**, is preferably conical and has sloping edges preferably at an angle of about 15° with respect to the longitudinal axis of the vice. Distal inner bore **27** at the level of the distal part **15** of the vice is enlarged in a preferably conical shape with sloping edges preferably at an angle of about 30° with respect to the longitudinal axis of the vice. Passage **21** can be formed to have different shapes than the ones depicted above. For example and without limitations, passage **21** can have a transverse section that is square or elliptical in one or several portions of central passage **21**. The edges of the side surfaces **22** at distal end **18** are optionally chamfered.

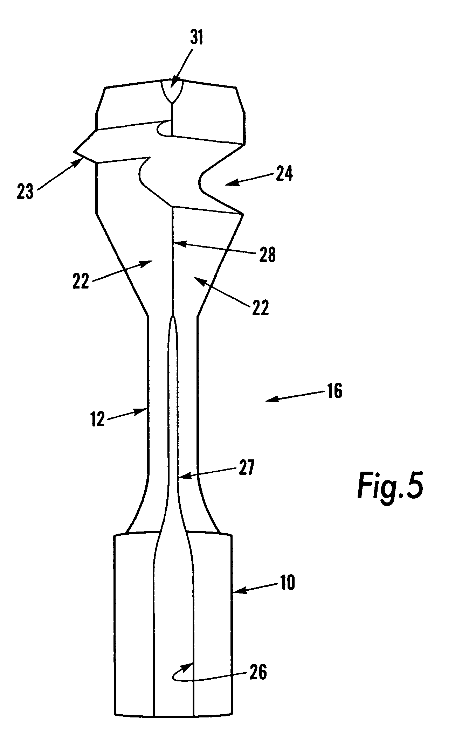

Figures 5 illustrates the inner side of a segment of the vice comprising an exemplary finger **16** and part of the base **10** from which finger **16** extends. The cut out view of the inner side of the central passage **21** illustrates the proximal portion of central passage **21** having preferably a conical bore **26**, continued distally by inner bore **27** of the intermediate portion of central passage **21** having preferably a cylindrical bore, in turn continued distally by the distal portion of central passage **21** which is delimited by the portion **28** of finger **16**. The diameter of the distal portion of central passage **21**, within the distal intermediate part **14** and head **11**, is critical to determine the maximum diameter of the elongated member that can be used with such a device. Because of the gripping action that is caused by urging together the fingers of the vice into an engagement position, the area of maximum grip, in the case of an elongated member that has the same diameter as the critical distal portion of central passage **21**, occurs between point **A** and point **B** as shown in Figure 4. When the elongated member has a diameter inferior to the diameter of the distal portion of central passage **21**, the maximum grip on the elongated member, when the fingers are in the engagement position, occurs at **A**. Depending on the amount of external force that is applied to the outer surface of the fingers at the engagement position, and depending on the material of which the fingers are made, the length of portion **28** that contacts the elongated member extends from point **A** to a point between **A** and **B**. Even for an elongated member having a diameter inferior to the diameter of the critical distal portion of central passage **21**, the entire distance **A** to **B** contacts the elongated member when the external force is sufficiently high and/or the material of which the fingers are made is sufficiently flexible. Referring back to Figure 5, the portion **28** is interrupted by recess **24**. Alternatively, it is contemplated that protuberance **23** and complementary recess **24** have a transverse dimension that is less than the transverse dimension of the side surface **22** of finger **16** at that level, to allow for a continuous portion **28.** It is also contemplated that portion **28,** which is linear in the preferred embodiment, be shaped to improve gripping of the elongated member.

Figure 6 depicts an alternative illustrative embodiment of the present invention wherein each finger **16** comprises several protuberances **23** and recesses **24** on one side surface **22** of a finger, to match complementary recesses **24** and protuberances **23** of an adjacent side surface, when the fingers **16** are urged together in an engagement position. The space so delimited between adjacent side surfaces has a double zigzag shape. It is to be understood that the shape of protuberances **23** and recesses **24** can vary to include, for example, hemispherical, spheroidal, cubical or other protuberances and respective complementary recesses. Moreover, although the protuberances and recesses are preferably located in the distal intermediate part **14** and head **11** of the vice, it is also within the scope of the present invention to form vices having protuberances and recesses which are located anywhere along the side surfaces **22** of each finger to form slits having either a single non-straight part or multiple non-straight parts that not contiguous.

In another embodiment of the present invention illustrated in Figure 7, the fingers are shaped to create slits that have a helical shape in the distal part **15**, head **11** and distal intermediate part **14** of the vice, thus creating a non-straight slit **19** which prevents elongated members to becoming entrapped between fingers **16** of the vice, when the fingers **16** are urged together in an engagement position.

The materials and methods of construction and the dimensions of the device are not essential and may vary. The material of which the vices are made is preferably, and not limited to, one of brass, stainless steel, copper, beryllium-copper alloy, or a polymer such as cellulose esters, polyesters, copolyesters, copolyester ethers (COPE) and polyethylene. Although it is preferable to use a single material to construct the vice in accordance of the present invention, it is within the scope of the present invention to construct vices either with a combination of materials, different parts of the vice being constructed from different materials, or with a mixture of at least two of the materials cited above. Furthermore, it is also to be understood that the vice of the present invention can be constructed for example from one single piece, or from several pieces constructed separately and subsequently joined, for example by pressing, bonding, welding or attaching the parts together in any other suitable way. Methods of manufacturing the metallic vices of the present invention include for example milling, electro-discharge machinery cutting, injection moulding or lathe turning. Alternatively, when for example injection moulding of a liquid crystal polymer is used, the device of the present invention is conveniently made from a number of pieces that are preferably identical, such as the piece illustrated in Figure 5, and that are subsequently joined to form the vice of the present invention. The injection moulding method of manufacture, by decreasing dramatically the production costs of the device, is well suited for industrial production of the present device. The dimensions of the vice are not essential and are given below solely as examples applying to the illustrative embodiments of the present invention.

| **Parameters** | **Approximate values in mm** |
|---|---|
| Total length of the vice | 12.50 |
| Length of base **10** | 3.50 |
| Length of [base **10** + neck **12**] | 7.90 |
| Diameter of base **10** | 3.20 |
| Depth of recess **24** | 0.90 |
| Length of distal part **15** | 0.53 |
| Width of slit **19** | 0.60 |

## Claims

1. A medical device for gripping an elongated member, wherein the device comprises a plurality of fingers (16), wherein the fingers have portions (28) between which the elongated member is to be gripped when the fingers are urged together into an engagement position, wherein each finger has a pair of side surfaces (22) each of which is to be urged towards a side surface of an adjacent finger when the fingers are urged into the engagement position, and wherein adjacent side surfaces are shaped with respect to one another resulting in a space (19) therebetween at least a part of which is non-straight to prevent penetration of said space (19) by the said elongated member.

2. A device as claimed in claim 1, wherein at least one of the spaces (19) has multiple non-straight parts which are non contiguous.

3. A device as claimed in any preceding claim, wherein adjacent side surfaces (22) have shapes with respect to one another that are not complementary.

4. A device as claimed in claim 1 or 2, wherein adjacent side surfaces (22) have shapes with respect to one another that are complementary.

5. A device as claimed in any preceding claim wherein adjacent side surfaces (22) of two fingers (16) have shapes with respect to one another that are identical to the shapes of at least one other pair of adjacent side surfaces.

6. A device as claimed in claim 1 or 2 comprising four fingers (16) and wherein diametrically opposed spaces (19) are identical.

7. A device as claimed in any of claims 1 to 4 wherein the shapes of adjacent side surfaces (22) with respect to one another are different in every pair of side surfaces.

8. A device as claimed in any one of the preceding claims, **characterised in that** the space (19) between adjacent side surfaces (22) of at least one pair has a single zigzag shape, a double zigzag shape or a helical shape.

9. A method of gripping an elongated member by clamping it within a device according to any one of the claims 1-8.

## Patentansprüche

1. Medizinische Vorrichtung zum Greifen eines langgestreckten Elements, wobei die Vorrichtung eine Vielzahl von Fingern (16) umfasst, wobei die Finger Abschnitte (28) aufweisen, zwischen denen das langgestreckte Element ergriffen werden soll, wenn die Finger in eine Eingriffsposition zusammengedrückt werden, worin jeder Finger ein Paar Seitenflächen (22) aufweist, die jeweils zu einer Seitenfläche eines benachbarten Fingers gedrückt werden sollen, wenn die Finger in die Eingriffsposition gedrückt werden, und worin benachbarte Seitenflächen so relativ zueinander geformt sind, dass ein Raum (19) dazwischen entsteht, von dem zumindest ein Teil nicht gerade ist, um Penetration des Raums (19) durch das langgestreckte Element zu vermeiden.

2. Vorrichtung nach Anspruch 1, worin zumindest einer der Räume (19) mehrere nicht-gerade Teile aufweist, die nicht angrenzend sind.

3. Vorrichtung nach einem der vorhergehenden Ansprüche, worin benachbarte Seitenflächen (22) Formen relativ zueinander aufweisen, die nicht komplementär sind.

4. Vorrichtung nach Anspruch 1 oder 2, worin benachbarte Seitenflächen (22) Formen relativ zueinander aufweisen, die komplementär sind.

5. Vorrichtung nach einem der vorhergehenden Ansprüche, worin benachbarte Seitenflächen (22) von zwei Fingern (16) Formen relativ zueinander aufweisen, die mit den Formen zumindest eines anderen Paars benachbarter Seitenflächen identisch sind.

6. Vorrichtung nach Anspruch 1 oder 2, umfassend vier Finger (16) und worin diametral gegenüberliegende Räume (19) identisch sind.

7. Vorrichtung nach einem der Ansprüche 1 bis 4, worin die Formen benachbarter Seitenflächen (22) relativ zueinander bei jedem Paar von Seitenflächen unterschiedlich sind.

8. Vorrichtung nach einem der vorhergehenden Ansprüche, **dadurch gekennzeichnet, dass** der Raum (19) zwischen benachbarten Seitenflächen (22) von zumindest einem Paar eine einzelne Zickzack-Form, eine doppelte Zickzack-Form oder eine spiralförmige Gestalt aufweist.

9. Verfahren zum Greifen eines langgestreckten Elements durch Einklemmen des Elements in einer Vorrichtung gemäß einem der Ansprüche 1-8.

## Revendications

1. Instrument médical pour la saisie d'un membre allongé, l'instrument comportant une pluralité de doigts (16), les doigts ayant des portions (28) entre lesquelles le membre allongé doit être saisi quand les doigts sont sollicités ensemble dans une position de prise, chaque doigt étant doté d'une paire de surfaces latérales (22), chacune de ces surfaces devant être sollicitée vers une surface latérale d'un doigt adjacent quand les doigts sont sollicités dans la position de prise, et les surfaces latérales adjacentes étant façonnées les unes par rapport aux autres, résultant en un espace (19) entre elles, dont au moins une partie n'est pas droite, pour empêcher ledit membre allongé de pénétrer à l'intérieur dudit espace (19).

2. Instrument selon la revendication 1, au moins un des espaces (19) ayant de multiples parties non droites qui ne sont pas contiguës.

3. Instrument selon l'une quelconque des revendications précédentes, les surfaces latérales adjacentes (22) ayant des formes les unes par rapport aux autres qui ne sont pas complémentaires.

4. Instrument selon la revendication 1 ou 2, les surfaces latérales adjacentes (22) ayant des formes les unes par rapport aux autres qui sont complémentaires.

5. Instrument selon l'une quelconque des revendications précédentes, les surfaces latérales adjacentes (22) de deux doigts (16) ayant des formes les unes par rapport aux autres qui sont identiques aux formes d'au moins une autre paire de surfaces latérales adjacentes.

6. Instrument selon la revendication 1 ou 2, comportant quatre doigts (16) et les espaces diamétralement opposés (19) étant identiques.

7. Instrument selon l'une quelconque des revendications 1 à 4, les formes des surfaces latérales adjacentes (22) les unes par rapport aux autres étant différentes dans chaque paire de surfaces latérales.

8. Instrument selon l'une quelconque des revendications précédentes, **caractérisé en ce que** l'espace (19) entre les surfaces latérales adjacentes (22) d'au moins une paire a une forme en simple zigzag, une forme en double zigzag ou une forme hélicoïdale.

9. Procédé de saisie d'un membre allongé en le serrant à l'intérieur d'un instrument selon l'une quelconque des revendications 1 à 8.
